## Europäisches Patentamt

## European Patent Office

## Office européen des brevets

(19)

(11) Veröffentlichungsnummer: **0 013 538 B1**

(12) **EUROPÄISCHE PATENTSCHRIFT**

(45) Veröffentlichungstag der Patentschrift:
06.04.83

(51) Int. Cl.³: **C 02 F 3/28**, E 03 F 5/20

(21) Anmeldenummer: 79810180.4

(22) Anmeldetag: 13.12.79

(54) Einrichtung zur Gewinnung von Methangas aus organischen Abfällen.

(30) Priorität: 03.01.79 AT 51/79
25.07.79 AT 5146/79

(43) Veröffentlichungstag der Anmeldung:
23.07.80 Patentblatt 80/15

(45) Bekanntmachung des Hinweises auf die Patenterteilung:
06.04.83 Patentblatt 83/14

(84) Benannte Vertragsstaaten:
BE CH DE FR GB IT LU NL SE

(56) Entgegenhaltungen:
CH-A 400 926
DE-C 806 655
FR-A-2 229 819
US-A-1 420 250
US-A-2 064 529

(73) Patentinhaber: Manahl, Robert, Dipl.-Ing., Bergstrasse 8,
A-6900 Bregenz (AT)

(72) Erfinder: Pfefferkorn, Herbert, Arlbergstrasse 101,
A-6900 Bregenz (AT)

(74) Vertreter: Werffeli, Heinz R., Dipl.-Ing., Postfach 245,
CH-8032 Zürich (CH)

### Einrichtung zur Gewinnung von Methangas aus organischen Abfällen

Die Erfindung bezieht sich auf eine Einrichtung zur Gewinnung von Methangas aus organischen Abfällen mit einem Gärraum, einem Gassammelraum und einem Nachgärraum, wobei der unmittelbar über dem Gärraum liegende Gassammelraum von dem Gärraum baulich nicht getrennt ist, sowie mit mindestens einer Zuleitung in den Gärraum und einer Ableitung für die überschüssige ausgegärte Flüssigkeit aus dem Nachgärraum, bei der die Räume wärmeisoliert, vorzugsweise zumindest zum Teil im Erdreich eingebettet sind und der Gärraum und der vorzugsweise oberhalb desselben angeordnete Nachgärraum nach Art kommunizierender Gefässe verbunden und vorzugsweise in einem Baukörper vereinigt sind.

Einrichtungen dieser Art gehören zum Stand der Technik. Beim Bau von Einrichtungen zur Gewinnung von Methangas muss den mikrobiologischen Gegebenheiten Rechnung getragen werden, um eine möglichst hohe Gasausbeute zu erzielen, dabei ist es wichtig, einmal dafür zu sorgen, dass nur so viel Frischschlamm der Einrichtung zugeleitet wird, dass der Gehalt der Gärmasse an flüchtigen Säuren unter einer bestimmten Rate bleibt, dass das Milieu alkalisch reagiert, dass für eine gleichmässige Temperatur unter Vermeidung von plötzlichen Temperaturstössen gesorgt wird und dass schliesslich das Entweichen des Gases nicht durch Schwimmdeckenbildung verhindert wird, mit anderen Worten, eine günstige Methanproduktion ist vor allem ein technisches Problem der Beschickung und Gestaltung der Gärräume sowie vor allem der Beseitigung der Schwimmdecke. Es ist eine Anlage bekannt geworden, bei der durch zeitweilige Drosselung der Gasabfuhr und der stetigen Gasentwicklung ein Überdruck erzeugt worden ist, der dazu benutzt wurde, die Schwimmdecke aufzulockern. Der Gärbehälter wird dabei durch zwei aussenstehende Schächte mit Füllgut beschickt. der Baukörper umfasst Gärraum, Gasbehälter und Faulschlammsilo. Der untere Teil des Baukörpers ist der Gärbehälter, dessen oberer Teil als Gasbehälter dient. Darüber liegt der Faulschlammbehälter, der als flacher Trichter mit seiner tiefsten Stelle durch einen senkrechten Kanal mit dem unteren Teil des Gärbehälters verbunden ist und der einen seitlichen Auslass für die überschüssige Gärflüssigkeit hat. Im oberen Teil des Gärbehälters oder im unteren Teil des Gasbehälters, beide Teile sind baulich nicht voneinander getrennt, ist ein Rechen angebracht. Das Füllgut wird durch den Einfüllschacht eingebracht und gelangt so in den unteren Teil des Gärbehälters. Das im Gärbehälter sich entwickelnde Gas steigt nach oben und sammelt sich im Gassammelraum an. Bei zunehmender Gasbildung entsteht ein Überdruck, der die Flüssigkeit aus dem Gärbehälter durch den senkrechten Schacht in den über dem Gasraum liegenden Schlammbehälter drückt. Sobald Gas entnommen wird, verringert sich der Druck im Gasbehälter, und die Flüssigkeit aus dem oben liegenden Faulschlammbehälter sinkt in dem senkrechten Schacht nach unten in den Gärraum zurück. Wird kein Gas entnommen, so steigt durch die stetige Gasentwicklung der Druck an und hebt die Flüssigkeit aus dem Gärbehälter wieder in den Faulschlammbehälter. Damit ist der Spiegel der Flüssigkeit im Gärbehälter im steten Steigen und Senken begriffen und auch natürlich die Schwimmdecke, die auf dem Flüssigkeitsspiegel aufliegt. Dabei soll durch den Rechen zwischen Gär- und Gasraum die Schwimmdecke so weit aufgelockert werden, dass sie das Gas in den Gasraum entweichen lässt. Hier wird also durch die zeitweilige Drosselung der Gasabfuhr und die stetige Gasentwicklung ein Überdruck erzeugt, der dazu benutzt wird, die Schwimmdecke aufzulockern und die ausgegasten Stoffe aus dem Gärsystem abzuführen. Diese Einrichtung dürfte sich jedoch – aus welchen Gründen immer – nicht bewährt haben, da ausschliesslich ein einziges Exemplar in dieser Bauart erstellt worden ist (Gewinnung und Verwertung von Methan aus Klärschlamm und Mist, Band III, Verlag Oldenburg 1956).

Es ist eine Vorrichtung zum Umwälzen von Schlamm in einem geschlossenen und mit einer absperrbaren Gasabzugsleitung versehenen Faulraum einer Kläranlage bekanntgeworden (DE-C-806 655). Diese besitzt einen innerhalb der Schwimmschlammzone und unterhalb dem Druck der Selbstkompression des Gases ausgesetzten Gassammelraums angeordneten, offenen Schwimmschlammtrichter. Dieser weist ein nach unten gerichtetes Rohr auf, dessen unteres Ende durch eine Rückschlagklappe verschliessbar ist. Durch das wiederholte Öffnen und Schliessen des Gasauslasshahnes in der Gasabzugleitung soll sich dieser Trichter mit Schwimmschlamm anfüllen und diesen unter der Druckwirkung des abgesperrten, anwachsenden Faulgasvolumens in den unteren Teil des Faulraumes ableiten. Dabei wird jedoch der in den Gassammelraum aufsteigende und wegen der Glockenform dieses Gassammelraumes sich verdichtende Schlamm durch das Hineindrücken in das aus dem Schwimmschlammtrichter nach unten führende Rohr noch weiter verdichtet, so dass in diesem Rohr bereits nach relativ kurzer Betriebszeit ein fest verdichtender Schlammtropfen steckt, der den weiteren Durchgang verhindert, so dass dieses Rohr des Schwimmschlammtrichters die ihm zugedachte Funktion nicht mehr ausüben kann mit der Folge, dass der sich im Gassammelraum immer mehr verdichtende Schwimmschlamm die weitere Gasproduktion bald unterbinden wird.

Bei einer anderen bekanntgewordenen Einrichtung dieser Art (US-A 2 064 529) sind zur Verhinderung der Bildung einer Schwimmschlammdecke mechanische Rührwerke vorgesehen. Solche mechanische Rührwerke stellen einen zusätzlichen Aufwand dar, der für einen wirtschaftlichen Betrieb einer solchen Anlage nicht zu rechtfertigen ist.

Eine andere bekanntgewordene Einrichtung zur Gewinnung von Methangas (US-A 1 420 250) weist zwei Behälter auf, wobei im ersten offenen als Sammelraum dienenden Behälter die anfallende Flüssigkeit gesammelt wird und von wo aus über eine aus diesem Behälter führende Leitung diese Flüssigkeit einem gasdicht verschliessbaren zweiten, als Gärraum dienenden Behälter zugeleitet wird. Dieser zweite Behälter ist mit einer mit einem Ventilverschluss ausgestatteten Gasabzugleitung versehen. Ferner ist eine weitere Leitung mit einem Ventil vorhanden, die aus diesem als Gärraum dienenden zweiten Behälter in den ersten Behälter zurückführt und die frei in diesen offenen Behälter mündet. Die abzubauende, zu vergärende Flüssigkeit rinnt aus dem Sammelbehälter über die ersterwähnte, mit einem Rückschlagventil versehene Leitung in den Gärraum, gast hier aus und drückt dabei bei geschlossener Gasabzugleitung die Flüssigkeit über die zweite Verbindungsleitung in den offenen Sammelbehälter zurück, welcher räumlich gesehen gegenüber dem Gärraum höher liegt. Ist das Flüssigkeitsniveau auf Grund des steigenden Gasdruckes im Gärraum auf die Höhe der Mündung dieser Rückführleitung abgesunken, so wird das Ventil in der Gasentnahmeleitung geöffnet, so dass das Gas abströmen kann, wodurch sich der Gärraum wiederum über die ersterwähnte Leitung mit dem Rückschlagventil füllen kann. Massnahmen, die die Entstehung der sich immer mehr verdichtenden Schwimmschlammdecke unterbinden, sind hier nicht vorgesehen.

Die Erfindung stellt eine unmittelbare Weiterentwicklung und Verbesserung dieser bekannten Einrichtungen dar, die darauf abzielt, diese Einrichtungen wirtschaftlich in grossem Umfang nutzen zu können, was voraussetzt, dass sie einerseits einen einfachen konstruktiven Aufbau besitzt und dass andererseits Voraussetzungen für ein selbsttätiges Arbeiten geschaffen werden, indem die Entstehung einer die Gasentwicklung behindernden Schwimmschlammdecke ohne besonderen maschinellen Aufwand unterbunden wird. Erfindungsgemäss gelingt dies dadurch, dass der obere Teil des Nachgärraumes einerseits über eine durch ein Ventil absperrbare Verbindungsleitung mit dem Gassammelraum und andererseits mit mindestens einer zu einem Druckausgleichbehälter führenden Gasentnahmeleitung verbunden ist und die Ableitung über einen hydraulischen, syphonartigen Verschluss mit dem Nachgärraum in Verbindung steht und durch das Ventil zeitabhängig und/oder in Abhängigkeit des Füllstandes im Gärraum und/oder des im Gassammelraum herrschenden Gasdruckes die Verbindungsleitung öffenbar oder schliessbar ist. Dank dieses Vorschlages ist das Heben und Senken der Schwimmdecke, das für deren wirkungsvolle Zerstörung notwendig ist, nicht mehr von der mehr oder weniger willkürlichen Gasabnahme abhängig, vielmehr sinkt und hebt sich die Schwimmdecke in einem Rhythmus, der ausschliesslich vom Ausmass der Gasentwicklung abhängig gemacht werden kann bzw. von diesem abhängig ist. Bei entsprechend grossem Einzugsgebiet wird die Anlage oder Einrichtung gleichmässig beschickt, so dass mit einer mehr oder weniger gleichmässigen Gasentwicklung über die Zeiteinheiten gerechnet werden kann, wogegen anderseits die Gasabnahme von gänzlich anderen Faktoren abhängig ist. Da der Baukörper für die Gewinnung von Methangas vornehmlich in landwirtschaftlich genutzten Gebieten zu errichten ist, ist es wichtig, dass dieser Baukörper einen relativ einfachen Aufbau hat, aus welchem Grunde nach einem weiteren Merkmal der Erfindung vorgeschlagen wird, dass der Nachgärraum seitlich des Gassammelraumes angeordnet ist und Nachgärraum und Gassammelraum niveaugleiche Decken aufweisen und die Verbindungsöffnung zum Gärraum und die Öffnung für die Ableitung an einander entgegengesetzten Enden des Nachgärraumes angeordnet sind.

Ein weiteres Merkmal der Erfindung, das zur Zerstörung der Schwimmdecke dient, wobei durch dieses Merkmal erreicht wird, dass die Schwimmdecke regelmässig von oben her benetzt wird, liegt darin, dass der Gassammelraum und der Nachgärraum durch eine Heberleitung miteinander verbunden sind und die Eintrittsmündung der Heberleitung im Bodenbereich des Nachgärraumes und die Austrittsmündung im oberen Bereich des Gassammelraumes angeordnet sind.

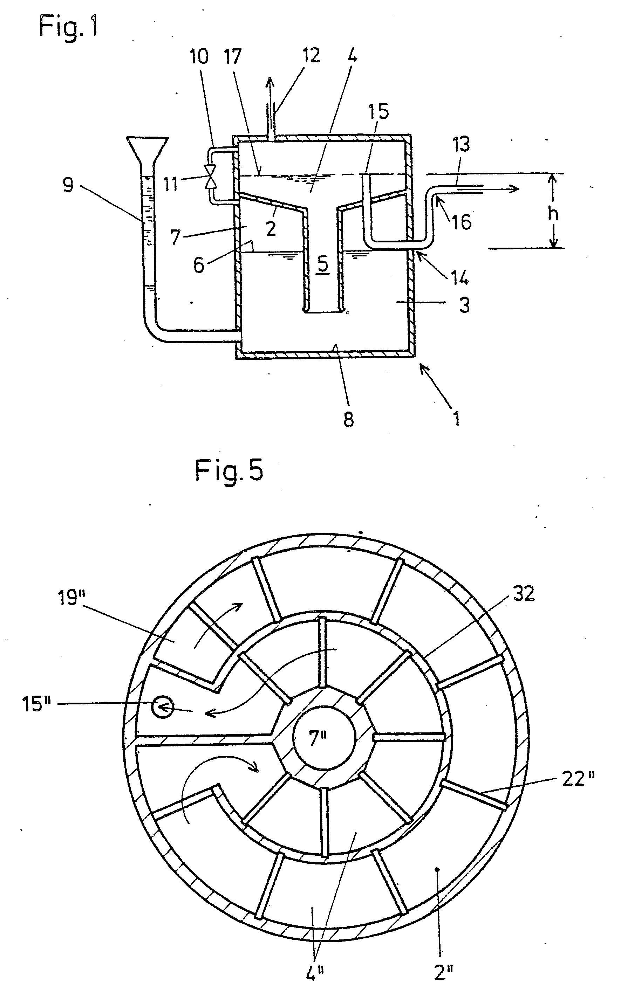

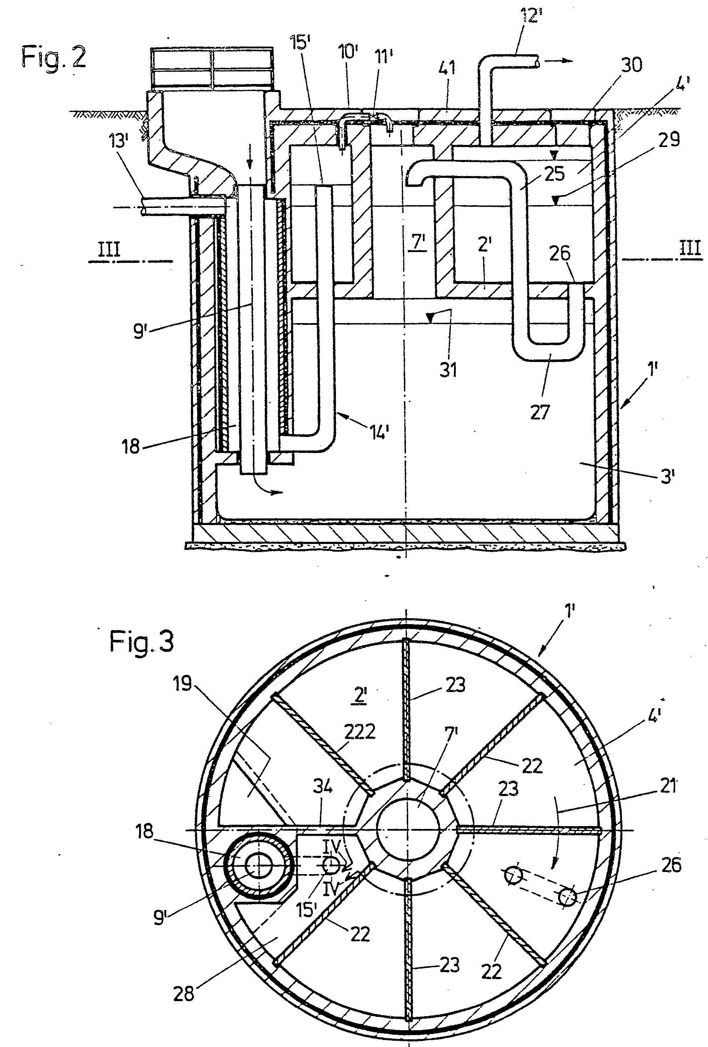

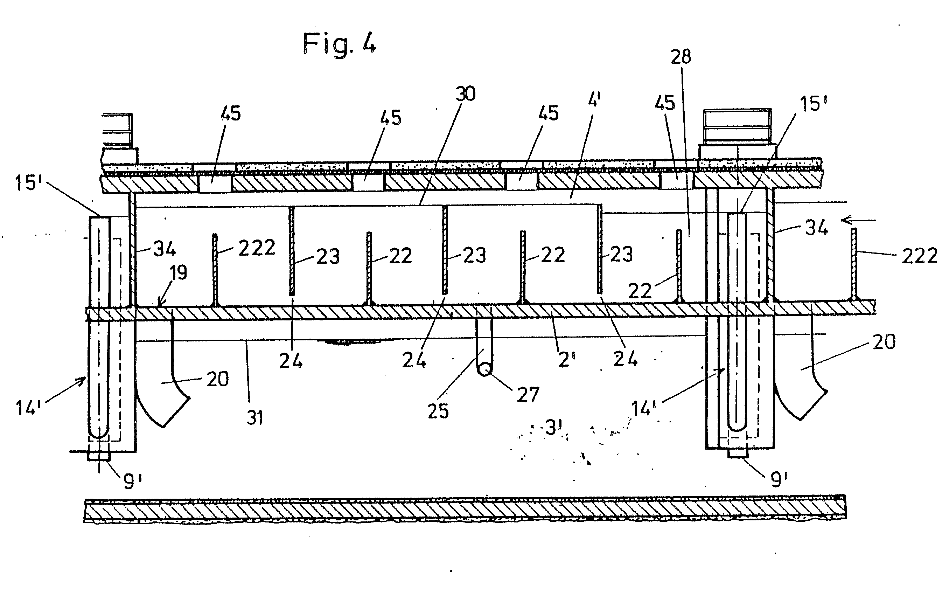

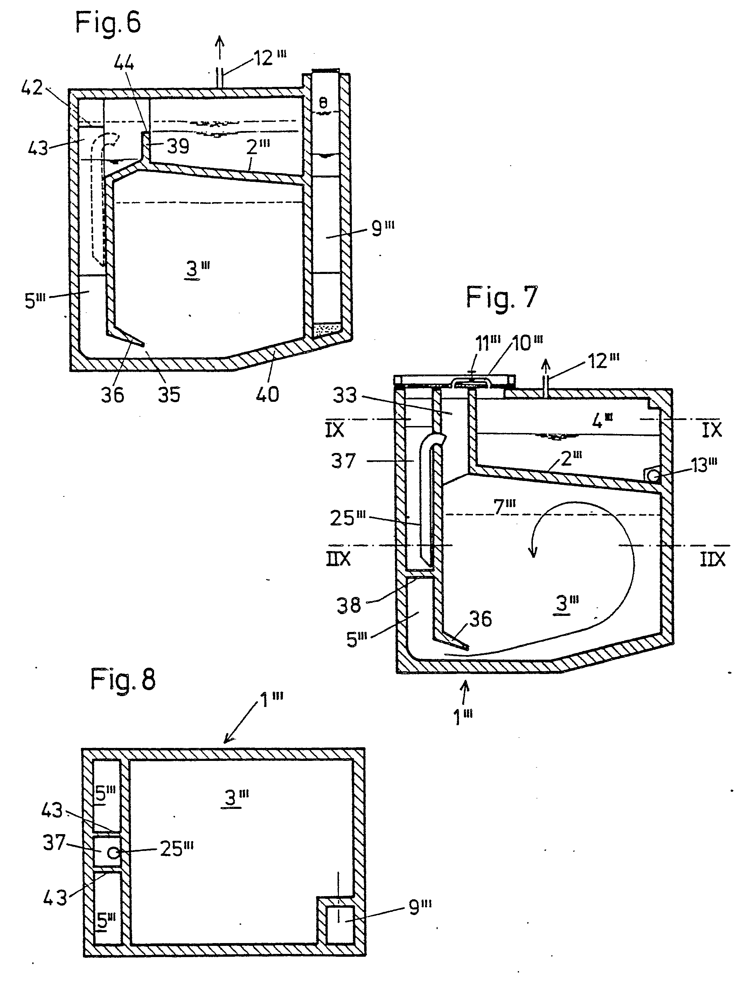

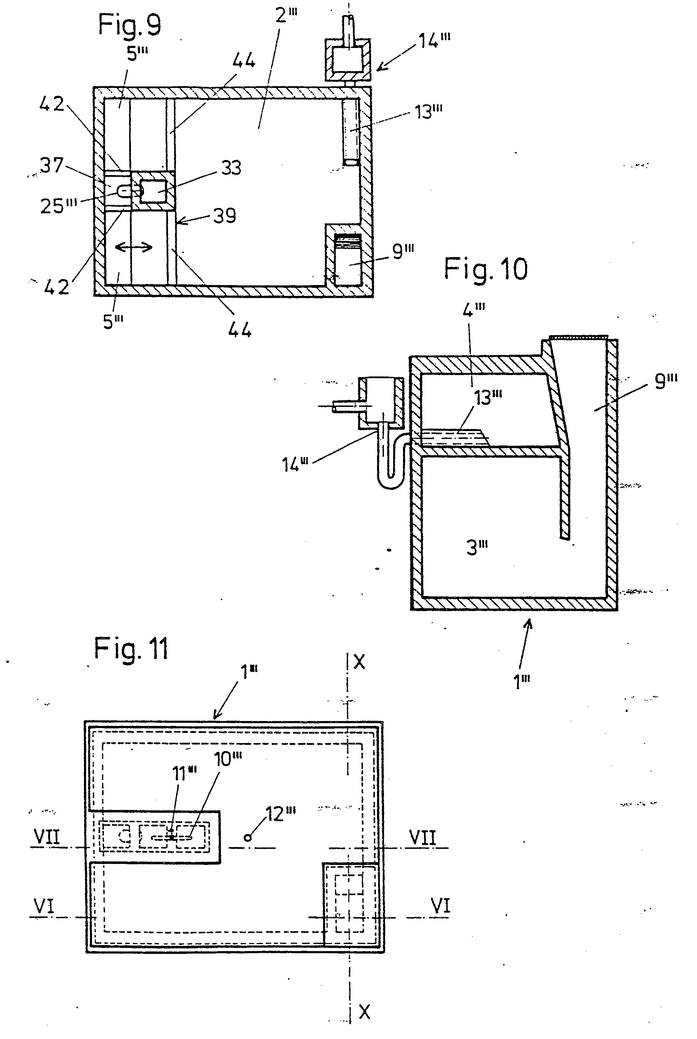

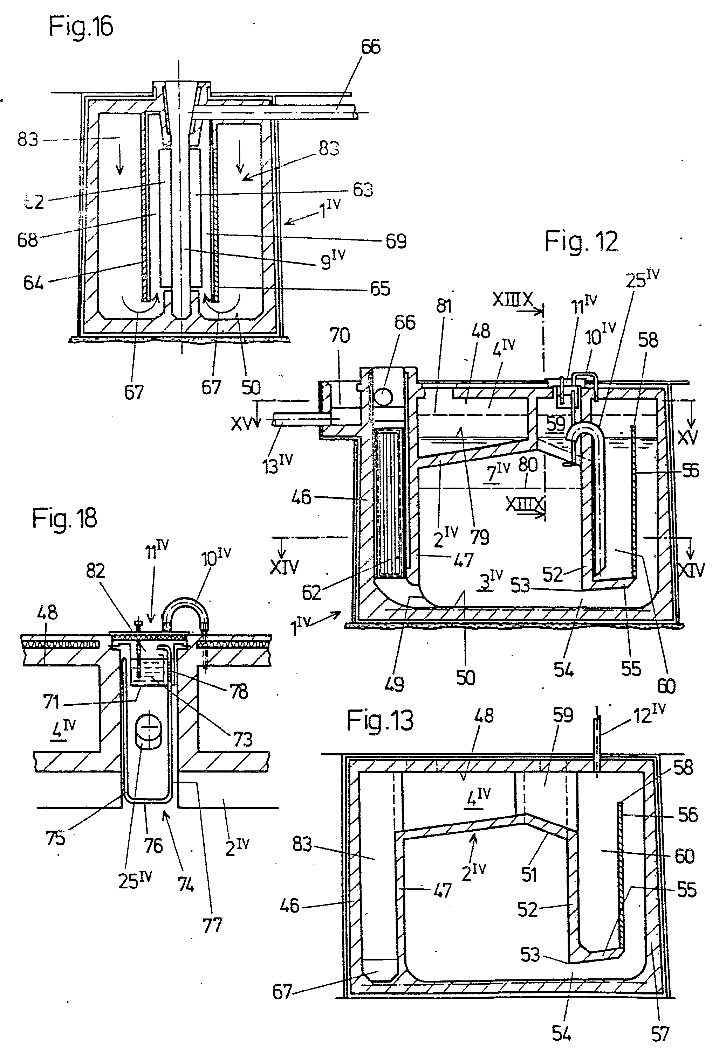

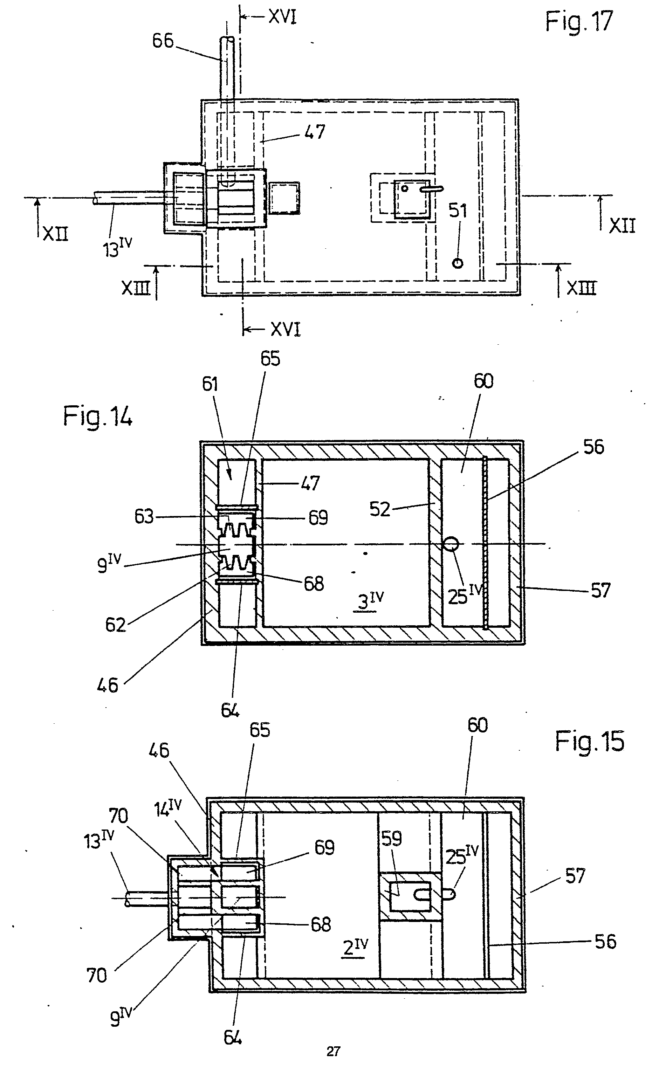

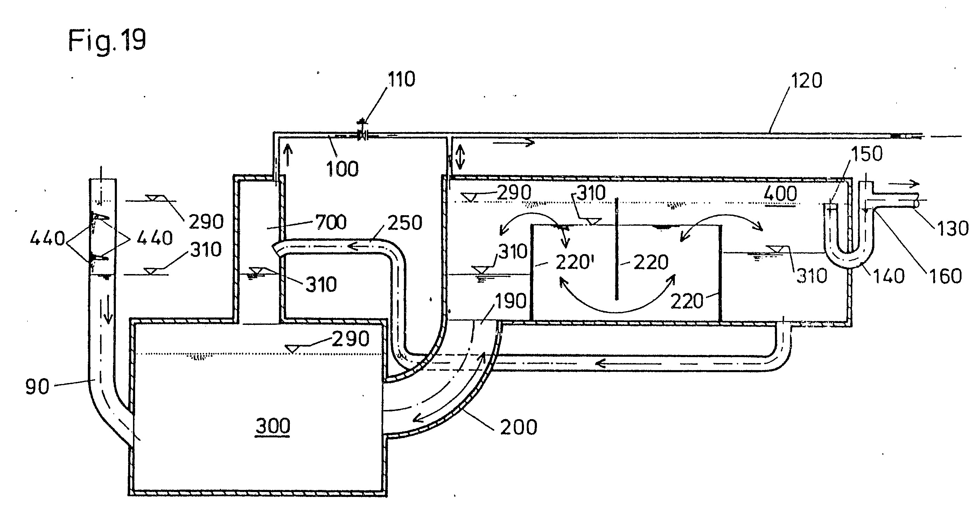

Anhand der Zeichnung werden Ausführungsbeispiele näher beschrieben und die daraus resultierenden Vorteile erläutert. Es zeigen: Fig. 1 einen Vertikalschnitt durch eine Einrichtung zur Gewinnung von Methangas in ihrem prinzipiellen Aufbau; Fig. 2 einen Vertikalschnitt durch eine zylindrische Anlage; Fig. 3 einen Horizontalschnitt nach der Linie III – III in Fig. 2 und Fig. 4 eine innere Abwicklung des in Fig. 2 gezeigten Baukörpers; Fig. 5 eine Ausgestaltungsvariante in Horizontalschnitt eines oberen Kreisringraumes nach Fig. 2; die Fig. 6–11 eine weitere Anlage, und zwar veranschaulichen Fig. 6 einen ersten Vertikalschnitt nach der Linie VI – VI in Fig. 11; Fig. 7 einen zweiten Vertikalschnitt nach der Linie VII – VII in Fig. 11; Fig. 8 einen Horizontalschnitt nach der Linie IX–IX in Fig. 7; Fig. 10 einen Vertikalschnitt nach der Linie IIX – IIX in Fig. 7; Fig. 9 einen Horizontalschnitt nach der Linie IX–IX in Fig. 7; Fig. 10 einen Vertikalschnitt nach der Linie X – X in Fig. 11 und Fig. 11 eine Draufsicht; ein weiteres Ausführungsbeispiel veranschaulichen die Fig. 12 bis 18, es zeigen: Fig. 12 einen mittleren Längsschnitt durch die Einrichtung nach der Schnittlinie XII – XII in Fig. 17; Fig. 13 einen zweiten Längsschnitt nach der Schnittlinie XIII – XIII in Fig. 17; Fig. 14 einen Horizontalschnitt nach der Linie XIV – XIV in Fig. 12; Fig. 15 einen zweiten Horizontalschnitt nach der Linie XV – XV in Fig. 12; Fig. 16 einen Querschnitt nach der Linie XVI – XVI in Fig. 17 und Fig. 17 eine Draufsicht auf die Einrichtung; Fig. 18 ein Detail aus Fig. 12 in vergrössertem Massstab nach der Schnittlinie XVIII – XVIII (Ventilautomatik); Fig. 19 zeigt den prinzipiellen Aufbau der in den Fig. 2 bis 18 gezeigten konstruktiven Lösungen, wobei hier in dieser Figur zum Zwecke der Veranschaulichung Gärraum und Nachgärraum als getrennte Baukörper dargestellt sind.

Fig. 1 stellt eine Einrichtung zur Gewinnung von Methangas im Vertikalschnitt dar, und zwar in ihrem prinzipiellen Aufbau. Ein zylindrischer Baukörper 1 mit einer vertikalen Achse, der wärmeisoliert ausgebildet ist und zusätzlich zumindest zum Teil beheizte Wandabschnitte aufweist, ist zur Gänze oder zumindest teilweise im Erdboden eingelassen, je nach den örtlichen Gegebenheiten, wo dieser Baukörper errichtet oder aufgestellt wird. Durch eine innere Zwischendecke 2 ist der Baukörper in zwei Räume unterteilt, nämlich einerseits den Gärraum 3 und Gassammelraum 7 und anderseits in den Nachgärraum 4. Gärraum 3 und Gassammelraum 7 sind voneinander baulich nicht getrennt. Ein hier zentral angeordneter vertikaler Schacht 5 verbindet die beiden Räume 3 und 4 nach Art kommunizierender Gefässe. Der Begriff kommunizierende Gefässe soll hier und im folgenden nur in der Weise verstanden werden, dass die in diesen beiden Räumen befindlichen Flüssigkeitsmengen die Möglichkeit haben, hin und her zu strömen, zumindest teilweise. Als Gärraum 3 wird hier und im folgenden jener Raum verstanden, der jeweils mit der auszugasenden Flüssigkeit gefüllt ist. Unmittelbar darüber liegt der Gassammelraum. Diese Räume ändern ihr Volumen während des Betriebes der Einrichtung, und es wird hier nochmals darauf verwiesen, dass diese beiden Räume baulich voneinander nicht getrennt sind. Nahe des Bodens 8 mündet der seitlich liegende Einfüllschacht 9, durch welchen die Abfälle eingebracht werden. Der Gassammelraum 7 und der obere Teil des Nachgärraumes 4 sind über eine Verbindungsleitung 10 miteinander verbunden. Diese Leitung ist durch ein Ventil 11 zu öffnen und zu schliessen. Eine weitere Leitung 12 führt aus dem oberen Teil des Nachgärraumes 4 zu einem hier nicht dargestellten Druckausgleichbehälter, beispielsweise zu einem Gasometer, so dass die Einrichtung stets gegen einen konstanten Betriebsdruck arbeitet. Eine Ableitung 13 führt die ausgegorene Flüssigkeit aus dem Nachgärraum 4, wobei zwischen Nachgärraum 4 und Leitung 13 ein syphonartiger, hydraulischer Verschluss 14 vorgesehen ist. Das Ventil 11 der Verbindungsleitung 10 sei hier zeitabhängig gesteuert. Es ist jedoch auch möglich, dieses Ventil 11 in Abhängigkeit des Gasdruckes im Gassammelraum 7 oder in Abhängigkeit von der jeweiligen Füllstandshöhe zu steuern. Die Einlauföffnung 15 der Ableitung 13 bzw. des hydraulischen Verschlusses 14 liegt im oberen Bereich des Nachgärraumes 4, und zwar in oder oberhalb der durch die Überlaufkante 16 des hydraulischen Verschlusses 14 vorgegebenen Horizontalebene.

Die Einrichtung arbeitet im Prinzip nun wie folgt, wobei davon ausgegangen wird, dass im Gärraum 3 und im Nachgärraum 4 Flüssigkeit vorhanden ist, dass das Ventil 11 geschlossen ist und dass im Gassammelraum 7 sich bereits ein solcher Druck aufgebaut hat, der der Wassersäule mit der Höhe h das Gleichgewicht zu halten vermag. Auch der Einfüllschacht 9 ist zum Teil gefüllt auf Grund seiner kommunizierenden Verbindung mit dem Gasraum 3. Auf das Niveau im Einfüllschacht 9 wirkt der äussere Luftdruck ein, so dass in diesem Einfüllschacht der Flüssigkeitsspiegel höher steht als im Gärraum 3. Die Füllmenge im Gärraum 3 scheidet weiterhin Gas aus. Im Gassammelraum 7 vergrössert sich damit weiter die Gasmenge und gleichzeitig der Gasdruck, der bewirkt, dass ein weiterer Teil der im Gärraum 3 noch vorhandenen Flüssigkeitsmenge nach oben durch den Verbindungsschacht 5 in den Nachgärraum 4 gedrückt wird. Dadurch sinkt der untere Flüssigkeitsspiegel 6 ab, und der obere Flüssigkeitsspiegel 17 steigt etwas an, wobei ein Teil der nach oben verdrängten und bereits ausgegorenen Flüssigkeit über die Ableitung 13 in einen hier nicht dargestellten Sammelraum rinnt. Der im Gassammelraum 7 entstehende Druck steuert das Ventil 11. Ist der Druck hinreichend hoch, wird das Ventil 11 geöffnet und der Druck im Gassammelraum 7 fällt plötzlich ab, wobei die Abfallgeschwindigkeit des Gasdruckes vom Querschnitt der Verbindungsleitung 10 abhängt. Es findet also zwischen dem Nachgärraum 4 und dem Gassammelraum 7 ein Druckausgleich statt. Dieser plötzliche Druckabfall im Gassammelraum 7 bewirkt nun, dass ein Teil der im oberen Nachgärraum 4 befindlichen Flüssigkeit durch den Vertikalschacht 5 nach unten stürzt, so dass im Gärraum 3 der Flüssigkeitsspiegel 6 wiederum ansteigt, wobei dies unter heftiger Turbulenz erfolgt, wogegen im oberen Nachgärraum 4 der Flüssigkeitsspiegel jedoch fällt. Dieser Vorgang läuft periodisch ab, wobei die Zeitdauer der einzelnen Perioden abhängig ist von der Heftigkeit und Intensität der Gasentwicklung im Gärraum 3. Durch dieses periodische Heben und Senken des Flüssigkeitsstandes im Gärraum 3 wird die Bildung einer Schwimmdecke weitgehend verhindert, wobei natürlich zusätzlich im unteren Raum 3 — 7 Brechkanten vorgesehen werden können, die auf die Schwimmdecke beim Steigen oder Senken derselben einwirken und diese mechanisch zerstören.

Der beschriebene Vorgang wiederholt sich, sobald das Ventil 11 geschlossen wird und sich im Gassammelraum 7 erneut ein Druck aufzubauen beginnt.

Eine erste bauliche Ausführungsvariante des in Fig. 1 gezeigten Prinzips veranschaulichen nun die Fig. 2, 3 und 4, wobei hier wie auch bei den folgenden Ausführungsbeispielen gleiche Teile mit gleichen Bezugsziffern ausgestattet werden, welchen zur Unterscheidung jeweils ein oder mehrere Indexstriche hinzugefügt werden.

Der Baukörper 1', auch hier wärmeisoliert mit beheizten Wandabschnitten und zumindest zum Teil im Erdboden eingelassen, ist durch einen vertikal stehenden Zylinder gebildet. Der Nachgärraum 4' ist hier durch eine kreisringförmige Kammer gestaltet, die den Gassammelraum 7' umschliesst. Die Zwischendecke 2' trennt hier den Gärraum 3' und den Nachgärraum 4'. Die Decke 41 des Baukörpers 2' grenzt sowohl den Gassammelraum 7' wie auch den Nachgärraum 4' nach oben ab. Der Einfüllschacht 9' ist hier in den Baukörper 1' integriert. Dieser Einfüllschacht 9' durchsetzt auf einen Teil seiner Länge ein nach aussen wärmeisoliertes Rohrstück 18, das wiederum einen Teil des U-förmigen syphonartigen, hydraulischen Verschlusses 14' darstellt, der nach aussen zur Ableitung 13' führt. Die Zwischendecke 2' besitzt eine Einlassöffnung 19, von wo aus ein Rohrstück 20 in den Gärraum 3' führt, was aus der Fig. 4 erkennbar ist. In Fig. 2 ist dieses Rohrstück durch die Rohre 9' und 18 verdeckt. In Durchflussrichtung (Pfeil, Fig. 3) gesehen, sind im kreisringförmigen Nachgärraum 4' voneinander distanzierte vertikale Wandscheiben 22 – 23 angeordnet, welche in wechselnder Folge mit dem Boden 2' verbunden bzw. von diesem Unter Bildung eines Spaltes 24 distanziert sind. Beim Ausführungsbeispiel, das hier gezeigt ist, ist auch eine Heberleitung 25 eingebaut, die den Gassammelraum 7' und den Nachgärraum 4' verbindet. Die Einlassöffnung 26 dieser Heberleitung 25 liegt in der Zwischendecke 2'. Von hier aus ist die Heberleitung 25 durch einen syphonartigen Krümmer 27 nach unten geführt und steigt dann wiederum an, um in den Gassammelraum 7' zu münden (Fig. 2). Zweckmässigerweise wird die Heberleitung so angeordnet, dass ihre Einlassöffnung 26 in der in Durchströmrichtung (Pfeil 21) gesehenen letzten Kammer 28 des Nachgärraumes 4' liegt, doch aus Gründen der Übersichtlichkeit ist hier beim gezeigten Ausführungsbeispiel (Fig. 2, 3 und 4) eine andere Anordnung gewählt worden. Der Gassammelraum 7' und der Nachgärraum 4' sind hier über die Verbindungsleitung 10' miteinander verbunden. In dieser Verbindungsleitung 10' ist das zeit-, volums- und/oder druckabhängig gesteuerte Ventil 11' angeordnet. Die Leitung 12' führt aus dem Nachgärraum zu einem nicht dargestellten Druckausgleichbehälter (Gasometer). Die Fig. 4 stellt einen Schnitt nach der Linie IV – IV in Fig. 3 dar, wobei die durch diesen Schnitt gewonnene Figur in die Zeichenebene abgewickelt ist. Der Massstab zu Fig. 4 ist gegenüber jenem nach den Fig. 2 und 3 verkleinert.

Wird davon ausgegangen, dass die Einrichtung gefüllt ist, sich jedoch noch kein Gas entwickelt habe, so ist der Füllstand mit der Niveaumarke 29 bezeichnet. Entwickelt sich in der Folge Methangas im Gärraum 3', so steigt dieses hoch, sammelt sich im Gassammelraum 7' und der dadurch entstehende Gasdruck drückt die Flüssigkeit im Gärraum 3' nach unten und die dermassen verdrängte Flüssigkeit steigt über das Rohrstück 20 nach oben in den Nachgärraum 4', und zwar so lange, bis die hier in Fig. 2 durch die Marken 30 bzw. 31 gegebenen Niveaustände erreicht worden sind. Das obere Niveau (Marke 30) liegt dabei oberhalb der Heberleitung 25, das untere Niveau (Marke 31) knapp über dem Krümmer 27. In der Kammer 28 des Nachgärraumes 4' ist das Niveau durch die Höhenlage der Einlauföffnung 15' bestimmt. Ein Teil der im Nachgärraum 4' befindlichen Flüssigkeit rinnt dabei über die Ableitung 13' nach aussen. Wird nun das Ventil 11' der Verbindungsleitung 10' geöffnet, so findet schlagartig ein Druckausgleich zwischen den beiden Räumen 3' und 4' statt, wobei ein Teil der im Nachgärraum 4' befindlichen Flüssigkeit durch die Bodenöffnung 19 und das Rohrstück 20 in den Gärraum 3' zurückfliesst, dabei für die entsprechende Verwirbelung und Durchmischung der Flüssigkeitsmenge im Gärraum 3' sorgt, ein anderer Teil der Flüssigkeit wird aus dem Nachgärraum 4' nunmehr über die aktiv gewordene Heberleitung 25 in den Gassammelraum 7 geleitet, von wo es von oben her auf die Schwimmdecke fällt, diese durchfeuchtet und gleichzeitig diese mit Bakterienstämmen aus dem Nachgärraum speist, so dass eine Art Impfvorgang stattfindet. Hat sich der Druckausgleich vollzogen, so wird das Ventil 11' wiederum geschlossen, die Gasentwicklung beginnt von neuem, und der beschriebene Vorgang wiederholt sich.

Die Anordnung, die hier anhand eines im Querschnitt runden Baukörpers gezeigt worden ist, lässt sich auch an einem quaderförmigen Baukörper realisieren. Aus den Fig. 3 und 4 ist ferner erkennbar, dass die Wandscheibe 34 den kreisringförmigen Nachgärraum 4' unterteilt, so dass dieser Kreisringraum eine endliche Begrenzung aufweist. Ferner sei darauf hingewiesen, dass die unmittelbar der bodenseitigen Eintrittsöffnung 19 benachbarte Zwischenwand 222 durch ihre Höhe das Ausmass derjenigen Flüssigkeitsmenge mitbestimmt, die beim Druckausgleich beim Öffnen des Ventils 11 wiederum in den Gärraum 3' zurückströmt. Durch mit Deckel verschlossene Schächte 45 ist der Nachgärraum 4' von oben zugänglich.

Beim gezeigten Ausführungsbeispiel ist ferner der Kreisringraum, der den Nachgärraum 4 bildet (siehe Fig. 3), über seine ganze Breite in jeweils einer Richtung durchströmt. Fig. 5 zeigt nun einen Horizontalschnitt, der jenem nach Fig. 3 entspricht, wobei jedoch dieser Kreisringraum durch eine Scheidewand 32 in zwei, in jeweils entgegengesetzte Richtungen durchströmte Durchflusszonen unterteilt werden kann. Die hier eingezeichneten Wandscheiben 22" sind mit Ihrer Unterkante vom Boden 2" und mit ihrer Oberkante von der Decke des Nachgärraumes 4" distanziert und darüber hinaus noch wechselweise schrägstehend angeordnet, so dass die im Falle des momentanen Druckausgleiches beim Öffnen des Ventils entstehende intensive Strömung die Flüssigkeit kräftig durchmischt. Fig. 5 stellt also eine Ausführungsvariante des Nachgärraumes nach Fig. 3 dar.

Die Fig. 6–11 zeigen nun eine weitere Ausführungsform, bei der der Baukörper 1"' im wesentlichen quaderförmig gestaltet ist. In diesem Baukörper 1"' bilden der Gärraum 3"' und der Gassammelraum 7"', der hier noch in einen Gasdom 33 übergeht, ein gemeinsames Volumen, das durch die schräg verlaufende Zwischendecke 2"' vom darüberliegenden Nachgärraum 4"' getrennt ist. Zwei stirnseitig vorgesehene Vertikalschächte 5"' verbinden den Gärraum 3"' und den Nachgärraum 4"' (Fig. 6). Die untere Mündungsöffnung 35 liegt im bodennahen Bereich des Gärraumes 3"' und führt über eine schräge äussere Schürze 36 zum eigentlichen vertikalen Schacht 5'''. Zwischen den beiden vertikalen Schächten 5''' ist ein sackartiger Schacht 37 vorgesehen (Fig. 7), der Teil des Nachgärraumes 4''' bildet und dessen Boden 38 unterhalb der Zwischendecke 2''' liegt. Die Oberkanten 42 der diesen sackartigen Schacht 37 begrenzenden Seitenwände 43 ist aus den Fig. 6 und 9 ersichtlich. In diesem sackartigen Schacht 37 ist eine Heberleitung 25''', deren eine Mündungsöffnung nahe dem Boden 38 dieses Schachtes 37 liegt und deren andere Öffnung in den Gasdom 33 ragt (Fig. 7). Im Scheitelbereich der schräg ansteigenden Zwischendecke 2''' ist eine Bordwand 39 vorgesehen, deren Oberkante 44 oberhalb des oberen Krümmers der Heberleitung 25''', jedoch unterhalb der Oberkante 42 der Wände 43 liegt. Der Einfüllschacht 9''' ist hier in den Baukörper 1''' integriert und in einer Ecke desselben angeordnet. Die Verbindungsleitung 10''' mit dem Druckausgleichsventil 11''' verbindet hier den oberen Bereich des Domes 33 mit dem oberen Bereich des Nachgärraumes 4''' (Fig. 7). Die Ableitung 13''' führt auch hier über einen hydraulischen syphonartigen Verschluss 14''' nach aussen. Der der Mündungsöffnung 35 der Vertikalschächte 5''' gegenüberliegende Teil 40 des Bodens des Gärraumes 3''' ist etwas schräg geneigt.

Die Funktionsweise der hier gezeigten Einrichtung ergibt sich im wesentlichen aus dem bereits Gesagten und soll hier nur der Vollständigkeit halber kurz erläutert bzw. ergänzt werden, wobei vorerst davon ausgegangen wird, dass das Ventil 11''' geschlossen ist, im Gärraum 3''' und im Nachgärraum 4''' gleicher Druck herrscht und der Nachgärraum 4''' und der Gärraum 3''' gefüllt sind, wobei das Füllniveau des Nachgärraumes 4''' durch die Oberkante 44 der Bordwand 39 gegeben ist. Beginnt nun die Füllmasse im Gärraum 3''' zu gasen, so sammelt sich im Dom 33 und anschliessend im Gassammelraum 7''' das Gas an, und der entstehende Druck presst die gasende Masse nach unten, der Spiegel im Gärraum 3''' sinkt ab und drückt gleichzeitig einen Teil der flüssigen Masse durch die beiden parallelen Vertikalschächte 5''' in den oben liegenden Gärraum 4''' bzw. nach Erreichung eines bestimmten Niveaus (Oberkante 42) in den Schacht 37 mit der Heberleitung 25''', wobei letzteres eintritt, wenn das obere Flüssigkeitsniveau die Differenz zwischen den Oberkanten 42 und 44 überfahren hat. Gleichzeitig wird auch ein Teil der überschüssigen, bereits abgearbeiteten Flüssigkeit nach aussen über die Ableitung 13''' abgeführt. Ist das Füllniveau des Gärraumes 3''' auf das vorgesehene Mass abgesunken und hat damit der Gasdruck im Gassammelraum 7''' bzw. im Dom 33 das vorgesehene Mass erreicht, so wird das Ventil 11''' geöffnet, wodurch der unter hohem Druck stehende Raum 33 mit dem Nachgärraum 4''' verbunden wird, wobei bei entsprechender Bemessung und Dimensionierung der Verbindungsleitung 10''' schlagartig im Gärraum der Druck abfällt. Aus dem Nachgärraum 4''' bzw. aus den vertikalen Schächten 5''' schiesst nun ein Teil der Gärmasse zurück in den Gärraum 3''', wobei durch die Gestaltung des Bodenteiles 40 und in Verbindung mit der Schürze 36 die hier lagernde Flüssigkeitsmasse intensiv durchmischt wird. Gleichzeitig wird dabei durch den abfallenden Flüssigkeitsspiegel und durch den abfallenden Druck im Dom 33 die Heberleitung 25''' aktiviert, so dass diese nun anspricht und die Flüssigkeit aus dem Schacht 37 in den Gassammelraum zurückführt, wobei sich diese Flüssigkeit von oben her auf die Schwimmdecke im Gärraum 3''' ergiesst und diese von oben her befeuchtet bzw. durchnässt. Da darüber hinaus ein Teil der Bakterienmenge aus dem Schacht 37 in den Gärraum 3''' rückgeführt wird, hat diese Heberleitung 25''' gleichzeitig eine sogenannte Impffunktion. Durch das wechselweise Heben und Senken der Schwimmdecke in Verbindung mit ihrer von oben her erfolgenden Durchnässung über die Heberleitung 25''' und ihr wiederholtes Anstossen an die schräg verlaufende Zwischendecke 2''' wird die Schwimmdecke aufgelockert und aufgebrochen bzw. aufgelöst oder aber überhaupt am Entstehen behindert. Nach dem erfolgten Druckausgleich und nach dem neuerlichen Schliessen des Ventiles 11''' steigt der Druck im Gärraum 3''' wiederum an, und der geschilderte Vorgang beginnt von neuem. Da die Gasableitung 12''' auch hier mit einem Druckausgleichbehälter (Gasometer) verbunden ist, arbeitet die Einrichtung stets gegen einen konstanten Arbeitsdruck, der vom Gasometer her bestimmt ist.

Eine weitere Ausführungsvariante veranschaulichen die Fig. 12 bis 18. Diese Einrichtung besteht aus einem Baukörper 1ᴵⱽ von im wesentlichen quaderförmiger äusserer Gestalt mit einem Gärraum 3ᴵⱽ, einem Gassammelraum 7ᴵⱽ und einem Nachgärraum 4ᴵⱽ. Benachbart der einen Stirnwand 46 liegt die Querwand 47, die in ihrem Mittelbereich bis zur oberen ebenen Decke 48 des Baukörpers 1ᴵⱽ hochgezogen ist, im unteren Mittelbereich jedoch eine Einlassöffnung 49 ausspart; zu beiden Seiten dieses Mittelbereiches jedoch gegenüber der Decke 48 abgesetzt ist und sich hier bis zum Boden 50 des Baukörpers 1ᴵⱽ hinunter erstreckt. In ca. zwei Drittel der Höhe dieser Querwand ist über die gesamte innere Breite des Baukörpers eine schräg ansteigende Zwischendecke 2ᴵⱽ vorgesehen, welche im Mittelbereich des Baukörpers 1ᴵⱽ in einen satteldachartig abfallenden Abschnitt 51 übergeht, der mit seinem Rand auf einer Wand 52 aufliegt, deren unterer Rand 53 zur Bildung einer über die Breite des Baukörpers 1ᴵⱽ reichenden schlitzförmigen Öffnung 54 vom Boden 50 des Baukörpers distanziert ist. Die Querwände 47 und 52 sowie die satteldachartige Zwischendecke 2ᴵⱽ begrenzen den Gärraum 3ᴵⱽ. An der unteren Kante 53 der Wand 52 ist eine vom Gärraum 3ᴵⱽ weg gerichtete, im wesentlichen waagrechte Schürze 55 vorgesehen, an deren äusseren Rand eine vertikale Trennwand 56 anschliesst, welche gegenüber der zweiten Stirnwand 57 des Baukörpers 1ᴵⱽ distanziert ist. Die Oberkante 58 der Trennwand 56 liegt unterhalb der waagrechten Baukörperdecke 48, jedoch etwas höher als die Firstkante der Zwischendecke 2ᴵⱽ. Der Deckenabschnitt 51 trägt einen Dom 59, der Teil des Gassammelraumes 7'ᵛ bildet und in welchen eine Heberleitung 25'ᵛ mündet und in welchem eine selbsttätig arbeitende Ventileinrichtung 11'ᵛ eingebaut ist, die im nachstehenden noch im Detail erläutert werden wird. Die Heberleitung 25'ᵛ liegt mit dem überwiegenden Teil ihrer Länge in der Kammer 60 und ist mit ihrer unteren Mündungsöffnung bis nahe zur waagrechten Schürze 55 geführt. Die Kammer 60 wird begrenzt durch die Schürze 55 und die Trennwand 56.

Die erste Stirnwand 46 des Baukörpers 1'ᵛ begrenzt mit der bereits erwähnten Querwand 47 eine Kammer 61, die durch innere Schottwände 62 und 63 und äussere Schottwände 64 und 65 wiederum unterteilt ist. Die inneren Schottwände 62 und 63, die vorzugsweise aus wärmeleitendem, chemisch beständigem Material, beispielsweise nicht rostendem Stahl gefertigt und profiliert sind, sind bis zum Boden 50 des Baukörpers 1'ᵛ geführt und bilden den Einfüllschacht 9'ᵛ, in welchen die Zuleitung 66 für den Frischschlamm mündet. Die äusseren Schottwände 64 und 65 sind von der Decke 48 aus nach unten geführt und begrenzen mit ihren unteren, vom Boden 50 distanzierten Kanten Durchflussöffnungen 67, die zu den vertikalen Schächten 68 und 69 führen, die zusammen mit den Überfallkanten 70 und der Ableitung 13'ᵛ einen hydraulischen Verschluss 14'ᵛ bilden. Die Überlaufkanten 70 sind zweckmässigerweise höhenverstellbar gelagert.

Als Teil der Ventileinrichtung 1'ᵛ ist im Dom 59 ein Topf 71 vorgesehen, der gegenüber dem Dom 59 und dem Gärraum 3'ᵛ abgedichtet ist und dessen Innenraum über die hier U-förmig geführte Leitung 72 mit dem Nachgärraum 4'ᵛ verbunden ist. Der Topf 71 ist zum Teil mit einer Flüssigkeit 73, beispielsweise Regenwasser gefüllt. Ein U-förmiges Rohr 74 liegt mit seinem einen Vertikalschenkel 75 und mit seinem waagrechten Abschnitt 76 im Dom 59, das obere Ende des anderen vertikalen Schenkels 77 hingegen ragt durch den Boden des Topfes 71 hindurch in diesen hinein, wobei die beiden Mündungen des Rohres 74 oberhalb des Flüssigkeitsspiegels im Topf 71 liegen. In dem von der Flüssigkeit 73 umspülten Abschnitt dieses Rohrstückes 77 sind Bohrungen 78 vorgesehen. Der Baukörper 1'ᵛ ist im Erdreich eingebettet, seine Wandungen sind isoliert, eventuell sogar beheizbar.

Die Einrichtung arbeitet nun wie folgt, wobei davon ausgegangen wird, dass ein Füllstand vom Niveau 79 vorgegeben ist (Fig. 1) und im Gärraum 3'ᵛ nunmehr die Gärung einsetzt. Das über die Zuleitung 66 und den Einfüllschacht 9'ᵛ und die bodennahe Einlassöffnung 49 eingebrachte Frischgut scheidet im Gärraum 3'ᵛ Gas aus, das sich im Dom 59 sammelt, wobei der sich hier entwickelnde Gasdruck so lange das Flüssigkeitsniveau im Gärraum 3'ᵛ nach unten drückt, wobei gleichzeitig das ausserhalb des Gärraumes 3'ᵛ liegende Niveau ansteigt (angedeutet durch die strichlierten Niveaulinien 80 und 81), bis die selbsttätig arbeitende Ventilmechanik 11'ᵛ anspricht. Im Ausgangsstadium der Betrachtung der Wirkungsweise (Niveau 79) ist das U-förmige Rohr 74 mit Flüssigkeit 73 aus dem Topf 71 gefüllt, da durch die Bohrungen 78 diese Flüssigkeit in dieses Rohr 74 rinnen kann, bis in den beiden kommunizierenden Schenkeln 75 und 77 Niveaugleichheit herrscht. Der im Gärraum 3'ᵛ ansteigende Gasdruck senkt nicht nur, wie schon erwähnt, das Niveau im Gärraum 3'ᵛ ab, er drückt gleichzeitig die im U-förmigen Rohr 74 befindliche Flüssigkeit in den Topf 74 zurück, bis das im Gassammelraum 7'ᵛ sich ansammelnde Gas über dieses U-förmige Rohr 74 in die vom Topf 71 begrenzte Kammer 82 strömt und von hier in den Nachgärraum 4'ᵛ über die Leitung 72 gelangt, wodurch zwischen den Räumen 3'ᵛ und 4'ᵛ der Druck ausgeglichen wird, so dass in der Folge im Gärraum 3'ᵛ das Niveau wiederum ansteigt, im Nachgärraum 4'ᵛ hingegen wiederum abfällt, bis in allen Räumen Druckgleichheit herrscht (Fig. 12). Durch das ansteigende Flüssigkeitsniveau im Nachgärraum 4'ᵛ wurde die Krümmung der Heberleitung 25'ᵛ überfahren, so dass beim Niveauabfall infolge des geschilderten Druckausgleiches aus der Kammer 60 Faulschlamm durch die Heberleitung 25'ᵛ gerissen wird und wiederum zurück in den Gärraum 3'ᵛ geführt wird. Durch den aus der Heberleitung 25'ᵛ herabfallenden Faulschlamm und das Bestreichen der schrägen Decke 2'ᵛ, durch das sich wechselweise hebende und senkende Niveau wird das Entstehen einer Schwimmdecke erfolgreich verhindert. Das im Gärraum 3'ᵛ befindliche Gut wird aber auch beim Niveau- und Druckausgleich durch die durch die Bodenöffnung 54 aus dem Nachgärraum 4'ᵛ rückfliessende Flüssigkeit in Turbulenz versetzt, was ebenfalls zur Vermeidung der Schwimmdeckenbildung beiträgt.

Die überschüssige Flüssigkeit, die auf der Decke 2'ᵛ sich ansammelt, strömt über die Firstkante der Decke 2'ᵛ in die vertikalen Schächte 83 und wird über die bodennahen Öffnungen 67 in die anschliessenden aufsteigenden Vertikalschächte 68 und 69 gedrückt, die einen syphonartigen, hydraulischen Verschluss bilden, von wo sie über die höhenverstellbaren Überlaufkanten 70 in die Ableitung 13'ᵛ gelangt. Das in den Schächten 68 und 69 ansteigende, warme Faulgut gibt einen Teil seiner Wärme über die inneren Schottwände 62 und 63 an das im Einfüllschacht 9'ᵛ befindliche Beschickungsgut ab, so dass dieses vorgewärmt in den Gärraum 3'ᵛ gelangt und so die Wärme des abzuführenden ausgefaulten Schlammes sinnvoll rückgeführt wird.

Das geschilderte Ansteigen und Sinken des Schlammniveaus spielt sich in steter Folge ab, ohne äussere Eingriffe, und die Frequenz dieses Wechsels hängt ab von der Art des Frischschlammes, der Grösse der Anlage und der herrschenden Temperaturen. Das im Nachgärraum 4'ᵛ sich sammelnde Biogas (Methangas) wird über eine Leitung 12'ᵛ in den nicht dargestellten Gasvorratsbehälter, insbesondere zu einem Gasometer geleitet.

Die Fig. 19 zeigt nun abschliessend den prinzipiellen Aufbau der in den Fig. 2 – 18 gezeigten konstruktiven Lösungen, wobei hier zum Zwecke der Veranschaulichung Gärraum 300 und Nachgärraum 400 als räumlich getrennte Baukörper dargestellt sind. Der Gärraum 300 geht in seinem oberen Teil in den turm- oder schachtartigen Gassammelraum 700 über. Der hier räumlich getrennte Nachgärraum 400 ist durch Wandscheiben 220 und 220' in einzelne Kammern unterteilt. Gärraum 300 und Nachgärraum 400 sind über das Rohrstück 200 nach Art kommunizierender Röhren verbunden. Die Mündungsöffnung dieses Rohrstükkes 200 im Bodenbereich des Nachgärraumes 400 ist mit 190 bezeichnet. Aus dem Nachgärraum führt über einen hydraulischen Verschluss 140 die Ableitung 130, wobei deren Einlassöffnung 150 im oberen Bereich des Nachgärraumes liegt, und zwar in oder oberhalb der durch die Überlaufkante 160 des hydraulischen Verschlusses 140 vorgegebenen Horizontalebene. Die Heberleitung 250 führt aus dem Nachgärraum 400 in den Gassammelraum 700, und zwar mit einem nach unten geführten Bogen zur Bildung eines hydraulischen Verschlusses. Über den Einfüllschacht 90 wird die Anlage beschickt. Hier in Fig. 19 ist noch gezeigt, dass im oberen Bereich des Einfüllschachtes 90 aus einer waagrechten Lage nach unten schwenkbare Arme 440 gelagert sind, die in ihrer waagrechten Lage bis ca. in die Mitte des Einfüllschachtes ragen. Die Leitung 100, in der das periodisch zu öffnende Ventil 110 angeordnet ist, verbindet den Gassammelraum 700 mit dem oberen Bereich des Nachgärraumes 400, von welchem aus auch die Leitung 120 zu einem Druckausgleichbehälter führt. Das Ausgleichsniveau 310 ist in den einzelnen Räumen eingetragen. Es wird erreicht, wenn die Anlage in Betrieb ist und wenn ein Druckausgleich stattgefunden hat. Das durch das Anwachsen des Gasdruckes im Gassammelraum 700 ansteigende bzw. absinkende Niveau ist durch die Hinweisziffer 290 angedeutet.

Ist die Anlage in Betrieb und wird vorausgesetzt, dass eben ein Gasaustausch bzw. Druckausgleich über das Ventil 110 und die Leitung 100 erfolgt sei, so ist das sich einstellende Niveau durch die Hinweisziffer 310 vorgegeben. Entwickelt sich nach Schliessung des Ventils 110 im Gärraum 300 Gas, so sammelt sich dieses im Gassammelraum 700 an und drückt den Wasserspiegel im Gärraum 300 nach unten, wobei gleichzeitig das Niveau im Nachgärraum 400 ansteigt, ebenso im Einfüllschacht 90, bis das Niveau 290 erreicht ist. Öffnet nun das Ventil 110, so gleicht sich die Druckdifferenz zwischen dem Gassammelraum 700 und dem Nachgärraum 400 aus mit der Folge, dass im Gärraum 300 der Flüssigkeitsspiegel wiederum ansteigt, im Nachgärraum 400 jedoch absinkt, wobei in der ersten Kammer des Nachgärraumes 400, die unmittelbar über der Öffnung 190 des Rohrstückes 200 liegt, der Spiegel weiter absinkt als in den nachfolgenden Kammern, bedingt durch die Höhe der ersten Trennwand 220'. Während dieses Druckausgleiches führt auch die Heberleitung 250 einen Teil der Flüssigkeitsmenge aus der letzten Kammer des Nachgärraumes 400 in den Gassammelraum 700 zurück, so dass auch in dieser letzten Kammer der Flüssigkeitsspiegel ebenfalls unter die oberste Kante der letzten Trennwand 220 absinkt. Nun schliesst wiederum das Ventil 110 und der beschriebene Vorgang beginnt von neuem.

Im Einfüllschacht 90 sind übereinander vorzugsweise mehrere Arme 440 schwenkbar angelenkt, welche in der Regel nach unten hängen, durch aufschwemmendes Gut jedoch in eine horizontale Lage gedrückt werden, welche sie nicht überschreiten können. Diese Arme ragen bis ca. in die Mitte des Einfüllschachtes und haben die Aufgabe, Festkörper, die in den Einfüllschacht gelangen, daran zu hindern, in diesem Einfüllschacht mit wechselweise steigendem und sinkendem Flüssigkeitsspiegel immer wieder anzusteigen und abzusinken. Werden nämlich solche Festkörper eingefüllt, so sinken sie mit fallendem Wasserspiegel im Einfüllschacht ab. Steigt nun mit dem anwachsenden Gasdruck im Gassammelraum 70 der Gasdruck an und drückt er dabei die Flüssigkeit wiederum im Einfüllschacht hoch, so kann die Flüssigkeit zwar hochsteigen, der bereits abgesunkene Festkörper wird jedoch an diesem Hochsteigen gehindert, da er beim Hochsteigen den schwenkbar gelagerten Arm in die Horizontale auslenkt und von diesem zurückgehalten wird, so dass er allmählich immer tiefer im Einfüllschacht absinkt und schlussendlich in den Gärraum 300 gelangt. Solche Rückhalteeinrichtungen können natürlich auch bei den Ausführungsbeispielen nach den Fig. 2 – 18 angeordnet werden, dort sind sie nicht dargestellt, um nicht die Übersichtlichkeit der Zeichnung zu beeinflussen.

Grundsätzlich wäre es ja auch möglich, die Einrichtung in der Weise zu bauen, wie dies die Zeichnung nach Fig. 19 veranschaulicht, also mit räumlich getrennten Gärräumen, doch ist eine solche Bauweise platzraubend, aus welchen Gründen in der Regel eine gedrängte Bauweise bevorzugt wird, wie dies die Fig. 1–18 veranschaulichen. Die Erfindung soll unbeschadet dessen aber auch eine solche Einrichtung erfassen, die räumlich getrennte Gärräume und Nachgärräume besitzt.

**Patentansprüche**

1. Einrichtung zur Gewinnung von Methangas aus organischen Abfällen, mit einem Gärraum, einem Gassammelraum und einem Nachgärraum, wobei der unmittelbar über dem Gärraum liegende Gassammelraum von dem Gärraum baulich nicht getrennt ist, sowie mit mindestens einer Zuleitung in den Gärraum und einer Ableitung für die überschüssige ausgegärte Flüssigkeit aus dem Nachgärraum, bei der die Räume wärmeisoliert, vorzugsweise zumindest zum Teil im Erdreich eingebettet sind und der Gärraum und der vorzugsweise oberhalb desselben angeordnete Nachgärraum nach Art kommunizierender Gefässe verbunden und vorzugsweise in einem Baukörper vereinigt sind, dadurch gekennzeichnet, dass der obere Teil des Nachgärraumes (4, 4', 4'', 4''', 4ᴵⱽ) einerseits über eine durch ein Ventil (11, 11', 11''', 11ᴵⱽ) absperrbare Verbindungsleitung (10, 10', 10''', 10ᴵⱽ) mit dem Gassammelraum (7, 7', 7'', 7''',

7''') und anderseits mit mindestens einer zu einem Druckausgleichbehälter führenden Gasentnahmeleitung (12, 12', 12''', 12''') verbunden ist und die Ableitung (13, 13', 13''', 13''') über einen hydraulischen, syphonartigen Verschluss (14, 14', 14''', 14''') mit dem Nachgärraum (4, 4', 4'', 4''', 4''') in Verbindung steht und durch das Ventil (11, 11', 11''', 11''') zeitabhängig und/oder in Abhängigkeit des Füllstandes im Gärraum (3, 3', 3''', 3''') und/oder des im Gassammelraum (7, 7', 7'', 7''', 7''') herrschenden Gasdruckes die Verbindungsleitung (10, 10', 10''', 10''') öffenbar oder schliessbar ist.

2. Einrichtung nach Anspruch 1, dadurch gekennzeichnet, dass der Nachgärraum (4', 4'', 4''') seitlich des Gassammelraumes angeordnet ist und Nachgärraum und Gassammelraum niveaugleiche Decken (41, 48) aufweisen und die Verbindungsöffnung (19, 54) zum Gärraum und die Öffnung (15, 67) für die Ableitung an einander entgegengesetzten Enden des Nachgärraumes angeordnet sind.

3. Einrichtung nach Anspruch 1, dadurch gekennzeichnet, dass im Nachgärraum (4') nahe der bodenseitigen Eintrittsöffnung (19) eine den Nachgärraum (4') teilende, mit dem Boden (2') desselben verbundene, jedoch nur über einen Teil der inneren Höhe derselben reichende Wandscheibe (222) vorgesehen ist.

4. Einrichtung nach Anspruch 1 oder Anspruch 2, dadurch gekennzeichnet, dass der Nachgärraum (4') durch in Durchflussrichtung aufeinanderfolgende vertikale Wandscheiben (22, 23) unterteilt ist, wobei diese Wandscheiben in wechselnder Folge mit dem Boden (2') des Nachgärraumes (4') verbunden bzw. von diesem unter Bildung eines Spaltes (24) distanziert sind.

5. Einrichtung nach einem der Ansprüche 1–4, dadurch gekennzeichnet, dass die dem Gärraum (3', 3''', 3''') zugewandte Mündungsöffnung (35, 54) der Gärraum und Nachgärraum verbindenden Leitung unterhalb des Bodens des Nachgärraumes liegt.

6. Einrichtung nach Anspruch 1, dadurch gekennzeichnet, dass der Gassammelraum und der Nachgärraum durch eine Heberleitung (25, 25''', 25''') miteinander verbunden sind und die Eintrittsmündung der Heberleitung im Bodenbereich des Nachgärraumes und die Austrittsmündung im oberen Bereich des Gassammelraumes angeordnet sind.

7. Einrichtung nach Anspruch 6, dadurch gekennzeichnet, dass die Heberleitung (25) von ihrer bodenseitigen Eintrittsöffnung (26) aus vorerst zur Bildung eines hydraulischen Verschlusses (27) bogenförmig nach unten geführt ist (Fig. 2).

8. Einrichtung nach Anspruch 6, dadurch gekennzeichnet, dass die Heberleitung (25''', 25''') in einer mit dem Nachgärraum (4''', 4''') verbundenen Kammer (37, 60) angeordnet ist, deren Boden (38, 55) unterhalb des Bodens (2''', 2''') des Nachgärraumes (4''', 4''') liegt (Fig. 7, Fig. 12).

9. Einrichtung nach einem der Ansprüche 1–8, dadurch gekennzeichnet, dass der Gärraum (3') als stehender Zylinder und der Nachgärraum (4') als darüberliegender Kreisringraum ausgebildet sind und der vom Kreisringraum ausgesparte Mittelteil mit dem Gärraum verbunden ist und als Gassammelraum (7, 7'') dient.

10. Einrichtung nach Anspruch 1, dadurch gekennzeichnet, dass mindestens ein Teil der Ableitung wärmeisoliert ausgebildet ist und ferner von der Zuleitung (9', 9''') in den Gärraum (3', 3''') durchsetzt ist (Fig. 2, Fig. 16).

11. Einrichtung nach Anspruch 1 oder 10, dadurch gekennzeichnet, dass die Einlauföffnung (15, 15') der Ableitung (13, 13') aus dem Nachgärraum (4, 4') im oberen Bereich desselben liegt, und zwar in oder oberhalb der durch die Überlaufkante (16) des hydraulischen Verschlusses (14, 14') vorgegebenen Horizontalebene (Fig. 1, Fig. 2).

12. Einrichtung nach Anspruch 9, dadurch gekennzeichnet, dass der als Kreisringraum ausgebildete Nachgärraum (4'') durch eine Mittelwand (32) in zwei in entgegengesetzten Richtungen durchflossene Kammern unterteilt ist (Fig. 5).

13. Einrichtung nach einem der vorstehenden Ansprüche 1 bis 12, dadurch gekennzeichnet, dass die Gärraum (3, 3''', 3''') und Nachgärraum (4, 4''', 4''') trennende Zwischendecke (2''', 2''') schräg verlaufend ausgebildet ist (Fig. 6, 7, 12).

14. Einrichtung nach Anspruch 1, dadurch gekennzeichnet, dass der Gärraum (3''') eine gegenüber der Decke (48) des Baukörpers (1''') zur Bildung eines Gassammelraumes (7''') tieferliegende, schräg verlaufende und in einen Dom (59) übergehende zweite, den Gärraum überspannende Decke (2''') aufweist und der seitlich des Gärraumes (3''') liegende Teil des Nachgärraumes (4''') mit diesem durch eine bodennahe Öffnung (54) verbunden ist und der Nachgärraum (4''') über eine einen hydraulischen, syphonartigen Verschluss aufweisende Ableitung (13''') verfügt und dass der Gassammelraum (7''') mit einer hydraulischen und durch den Druck des gebildeten Gases betätigbaren, das Gasvolumen taktweise vom Gassammelraum (7''') in den Nachgärraum (4''') überführenden und gleichzeitig ein portionsweises Abführen des Faulschlammes über einen Heber (25''') auslösenden Ventileinrichtung (11''') verbunden ist.

15. Einrichtung nach Anspruch 14, dadurch gekennzeichnet, dass sich die bodennahe Öffnung (54) schlitzartig über die Breite des Baukörpers (1''') erstreckt und vom oberen Rand (53) dieser Öffnung gegen den Nachgärraum (4''') sich eine im wesentlichen waagrechte Schürze (55) erstreckt, von deren Rand aus sich gegen die Decke (48) des Baukörpers (1'''), jedoch vor dieser unter Bildung eines Spaltes endend, eine im wesentlichen vertikale Trennwand (56) erstreckt (Fig. 12).

16. Einrichtung nach Anspruch 15, dadurch gekennzeichnet, dass in einer von der Trennwand (56) und der den Gärraum (3''') gegenüber dem Nachgärraum (4''') begrenzenden Wand (52) gebildeten Kammer (60) die bis zur Schürze (55) reichende und in den Dom (59) des Gassammelraumes (7''') mündende Heberleitung (25''') angeordnet ist.

17. Einrichtung nach den Ansprüchen 15 oder 16, dadurch gekennzeichnet, dass die Oberkante (58) der Trennwand (56) um ein geringes Mass oberhalb der Überlaufkante · der Heberleitung (25''') liegt.

18. Einrichtung nach den Ansprüchen 1 bis 14, dadurch gekennzeichnet, dass die eine den Gärraum (3''') im Baukörper (1''') begrenzende, die Einlassöffnung (49) für den Abfall aufweisende Querwand (47) mit einer Stirnwand (46) des Baukörpers (1''') eine Kammer (61) bildet, welche durch mindestens vier, vorzugsweise symmetrisch zur Baukörpermittelebene angeordnete Schottwände (62, 63, 64, 65) unterteilt ist, wobei die inliegenden Schottwände (62, 63) bis zum Boden (50) des Baukörpers (1''') geführt sind und den Einfüllschacht (9''') bilden und die beiden aussenliegenden Schottwände (64, 65) zur Bildung von Durchflussöffnungen (67) vom Boden (50) des Baukörpers (1''') abgesetzt sind (Fig. 14, 16).

19. Einrichtung nach Anspruch 18, dadurch gekennzeichnet, dass die von den aussenliegenden Schottwänden (64, 65) begrenzten Schächte (68, 69) in ihrem oberen Bereich über vorzugsweise höhenverstellbare Überfallkanten (70) in die Ableitung (13''') münden.

20. Einrichtung nach Anspruch 18, dadurch gekennzeichnet, dass die inliegenden Schottwände (62, 63) aus vorzugsweise profilierten Metallblechen gebildet sind.

21. Einrichtung nach Anspruch 14, dadurch gekennzeichnet, dass die im Dom (59) des Gassammelraumes (7''') vorgesehene Ventileinrichtung (11''') aus einer teilweise mit Flüssigkeit (73) gefüllten topfartigen Kammer (82) besteht, die über eine Rohrleitung (10''') mit dem Nachgärraum (4''') verbunden ist und ein U-förmiges Rohrstück (74) mit seinem einen Schenkel (75) im oberen Bereich des Domes (59) liegt und der andere Schenkel (77) in die topfartige Kammer (82) ragt, wobei die Mündungsöffnung (84) oberhalb des Flüssigkeitsspiegels liegt und in dem von der Flüssigkeit (73) umspülten Abschnitt des Schenkels (77) Bohrungen (78) vorgesehen sind (Fig. 18).

22. Einrichtung nach Anspruch 21, dadurch gekennzeichnet, dass der die beiden vertikalen Schenkel (75, 77) des U-förmigen Rohrstückes (74) verbindende Abschnitt (76) im wesentlichen waagrecht verläuft und vorzugsweise länger ist, als es dem Abstand der Schenkel (75, 77) entspricht.

23. Einrichtung nach einem der vorstehenden Ansprüche 1 bis 22, dadurch gekennzeichnet, dass im Einfüllschacht (90) vorzugsweise in dessen oberem Bereich aus einer horizontalen Lage nach unten schwenkbare Arme (440) vorgesehen sind, welche zweckmässigerweise in ihrer Horizontallage zirka bis in die Mitte des Einfüllschachtes (90) ragen und vorzugsweise mehrere solcher Arme über die Höhe des Einfüllschachtes angeordnet sind (Fig. 19).

## Claims

1. Plant for obtaining methane gas from organic waste, with a fermentation chamber, a gas-collection space and a post-fermentation chamber, in which the gas-collection space lying above the fermentation chamber ist not separated structurally from the fermentation chamber, and also with at least one feed duct into the fermentation chamber and one discharge duct for the excess fermented liquid from the post-fermentation chamber, wherein the chambers are heat insulated, are preferably embedded at least partly in the ground and the fermentation chamber and the postfermentation chamber, which is preferably arranged above the former, are connected in the manner of communicating vessels and, preferably, are combined into a unit, characterised in that the upper part of the post-fermentation chamber (4, 4', 4'', 4''', 4''') is connected, on the one hand, via a connecting duct (10, 10', 10''', 10''') closable by a valve (11, 11', 11''', 11''') to the gas-collection space (7, 7', 7'', 7''', 7''') and, on the other hand, to at least one gas-take off duct (12, 12', 12''', 12'''), and the discharge duct (13, 13', 13''', 13''') is communicated with the post-fermentation chamber (4, 4', 4''', 4''') via a liquid-seal trap and the connecting duct (10, 10', 10''', 10''') can be opened or closed by the valve (11, 11', 11''', 11''') time-dependently and/or depending on the level of filling in the fermentation chamber (3, 3', 3''', 3''') and/or gas pressure prevailing in the gas-collection space (7, 7', 7'', 7''', 7''').

2. Plant according to claim 1, characterised in that the post-fermentation chamber (4', 4'', 4''') is arranged laterally of the gas-collection space and the post-fermentation chamber and gas-collection space have ceilings (41, 48) at the same level, and the connecting aperture (19, 54) to the fermentation chamber and the aperture (15, 67) for the discharge duct are disposed at opposite ends of the post-fermentation chamber.

3. Plant according to claim 1, characterised in that in the post-fermentation chamber (4') near the inlet aperture (19) on the bottom is provided a wall plate (222) which divides the post-fermentation chamber (4') which is connected to the bottom (2') thereof but which extends only over a part of the internal height thereof.

4. Plant according to claim 1 or claim 2, characterised in that the post-fermentation chamber (4') is divided up by vertical wall plates (22, 23) succeeding one another in throughflow direction, these wall plates being connected in alternating sequence to the bottom (2') of the post-fermentation chamber (4') and being spaced therefrom forming a gap (24).

5. Installation according to any of claims 1 to 4 characterised in that the outlet opening (35, 54) facing the fermentation chamber (3', 3''', 3''') of the duct connecting the fermentation chamber and post-fermentation chamber, lies below the bottom of the post-fermentation chamber.

6. Plant according to claim 1, characterised in that the gas-collection space and the post-fermentation chamber are connected to one another by a siphon duct (25, 25''', 25''') and the inlet opening of the siphon duct ist arranged in the bottom zone of the post-fermentation chamber and the outlet opening is arranged in the upper zone of the gas-collection space.

7. Plant according to claim 6, characterised in that starting from its inlet opening (25) on the bottom the siphon duct (25) is initially directed downwards in curved manner to form a liquid-seal trap (27) (Fig. 2).

8. Plant according to claim 6, characterised in that the siphon duct (25''', 25''') is arranged in a chamber (37, 60) which is connected with the post-fermentation chamber (4''', 4'') and of which the bottom (38, 55) lies below the bottom (2''', 2'') of the post-fermentation chamber (4''', 4'')(Fig. 7, Fig. 12).

9. Plant according to any of claims 1 to 8 characterised in that the fermentation chamber (3') is designed as an upright cylinder and the post-fermentation chamber (4') is designed as an annular chamber situated thereover, and the centre part set apart from the annular chamber is connected with the fermentation chamber and serves as a gas-collection space (7, 7').

10. Plant according to claim 1, characterised in that at least one part of the outflow duct is of heat-insulated design and, in addition, is penetrated by the feed duct (9', 9'') in the fermentation chamber (3', 3'')(Fig. 2, Fig. 16).

11. Plant according to claim 1 or claim 10, characterised in that the intake aperture (15, 15') of the outflow duct (13, 13') from the post-fermentation chamber (4, 4') is situated in the upper zone thereof, namely in or above the horizontal plane defined by the overflow edge (16) of the liquid-seal trap (14, 14')(Fig. 1, Fig. 2).

12. Plant according to claim 9 characterised in that the post-fermentation chamber (4'') designed as an annular chamber is divided up by a centre wall (32) into two chambers through which flow takes place in opposite directions (Fig. 5).

13. Plant according to any of the preceding claims 1 to 12, characterised in that the intermediate ceiling (2'', 2'') separating the fermentation chamber (3, 3''', 3'') and post-fermentation chamber (4, 4''', 4'') is designed to extend obliquely (Figs. 6, 7, 12).

14. Plant according to claim 1, characterised in that the fermentation chamber (3'') has an obliquely extending second ceiling (2'') which is situated lower down in relation to the ceiling (48) of the unit (1'') so as to form a gas-collection space (7''), which merges into a dome (59) and which spans the fermentation chamber, and the part of the post-fermentation chamber (4'') situated laterally of the fermentation chamber (3'') is connected therewith through an opening (54) near the bottom and the post-fermentation chamber (4'') is provided with an outflow duct (13'') having a liquid-seal trap, and in that the gas-collection space (7'') is connected with a hydraulic valve device (11'') which is actuable by the pressure of the gas formed, which cyclically transfers the gas volume from the gas-collection space (7'') into the post-fermentation chamber (4'') and which, at the same time, triggers a discharge in portions of the sludge via a siphon duct (25'').

15. Plant according to claim 14, characterised in that the opening (54) near the bottom extends slot-like over the width of the unit (1'') and from the upper edge (53) of this opening a substantially horizontal apron (55) extends towards the post-fermentation chamber (4''), a substantially vertical dividing wall (56) extending from the edge of this apron towards the ceiling (48) of the unit (1'') but terminating before this to form a gap (Fig. 12).

16. Plant according to claim 15, characterised in that in a chamber (60), which is formed by the dividing wall (56) and by the wall (52) bounding the fermentation chamber (3'') with respect to the post-fermentation chamber (4''), is arranged the siphon duct (25'') extending as far as the apron 55 and opening into the dome (59) of the gas-collecting space (7'').

17. Plant according to claims 15 or 16, characterised in that the upper edge (58) of the dividing wall (56) is situated slightly above the overflow edge of the siphon duct (25'').

18. Plant according to claims 1 to 14, characterised in that a traverse wall (47) which bounds the fermentation chamber (3'') in the unit (1'') and which has the inlet opening (19) for the waste, forms with an end wall (46) of the unit (1'') a chamber (61) which is divided up by at least four partition walls (62, 63, 64, 65) the innerlying partition walls (62, 63) being directed up to the bottom (50) of the unit (1'') and forming the feed chute (9'') and the two outerlying partition walls (64, 65) being spaced from the bottom (50) of the unit (1'') so as to form throughflow openings (67) (Figs. 14, 16).

19. Plant according to claim 18, characterised in that the shafts (68, 69) bounded by the outerlying partition walls (64, 65) open into the outflow duct (13'') in their upper zone via preferably vertically adjustable overflow edges (70).

20. Plant according to claim 18 characterised in that the innerlying partition walls (62, 63) are formed from preferably profiled metal sheets.

21. Plant according to claim 14, characterised in that the valve device (11'') provided in the dome (59) of the gas-collection space (7'') comprises a cup-like chamber (82) which is partly filled with liquid (73) and which is connected with the post-fermentation chamber (4'') via a (pipe 10'') and a U-shaped length of pipe (74) is situated with one of its legs (75) in the upper zone of the dome (59) and the other leg (77) projects into the cup-like chamber (82), the outlet opening (84) lying above the surface of the liquid and bores (78) being provided in the portion of the leg (77) surrounded by the liquid (73)(Fig. 18).

22. Plant according to claim 21 characterised in that the portion (76) connecting both vertical legs (75, 77) of the U-shaped length of pipe (74) extends substantially horizontally and, preferably, is longer than corresponds to the distance apart of the legs (75, 77).

23. Plant according to any of the preceding claims 1 to 22, characterised in that in the feed chute (90) preferably in its upper region, arms (440) are provided which can pivot downwards from a horizontal position and which, advantageously, in their horizontal position project approximately as far as into the centre of the feed chute (90) and, preferably, a plurality of such arms are arranged over the height of the feed chute (Fig. 19).

## Revendications

1. Installation pour la récupération de méthane gazeux à partir de déchets organiques comprenant une chambre de fermentation, une chambre collectrice de gaz et une chambre de post-fermentation, la chambre collectrice de gaz disposée directement au-dessus de la chambre de fermentation n'étant pas par construction séparée de celle-ci, ainsi qu'au moins une conduite d'amenée dans la chambre de fermentation et une conduite d'évacuation pour le liquide en excès hors de la chambre de post-fermentation, installation dans laquelle les chambres sont isolées thermiquement, et de préférence au moins en partie enfouies dans le sol, la chambre de fermentation et la chambre de post-fermentation disposée de préférence au-dessus d'elle étant reliées entre elles, à la manière de vases communicants, en un même corps de construction, installation caractérisée en ce que la partie supérieure de la chambre de post-fermentation (4, 4', 4'', 4''', 4'v) étant reliée, d'une part, par une conduite de liaison (10, 10', 10''', 10'v) obturable par une soupape (11, 11', 11''', 11'v) avec la chambre collectrice de gaz (7, 7', 7'', 7''', 7'v), et, d'autre part, à au moins une conduite de prélèvement de gaz (12, 12', 12''', 12'v) conduisant à un récipient d'équilibrage de pression, la conduite d'évacuation (13, 13', 13''', 13'v) étant en liaison, par l'intermédiaire d'une fermeture hydraulique du genre siphon (14, 14', 14''', 14'v) avec la chambre de post-fermentation (4, 4', 4'', 4''', 4'v), et la conduite de liaison (10, 10', 10''', 10'v) pouvant être ouverte ou fermée par la soupape (11, 11', 11''', 11'v) en dépendance du temps, et/ou de l'état de remplissage de la chambre de fermentation (3, 3', 3''', 3'v), et/ou de la pression de gaz régnant dans la chambre collectrice de gaz (7, 7', 7'', 7''', 7'v).

2. Installation suivant la revendication 1, caractérisée en ce que la chambre de post-fermentation (4', 4'', 4'v) est disposée sur le côté de la chambre collectrice de gaz, ces deux chambres présentant des plafonds (41, 48) à même niveau, et l'ouverture de liaison (19, 54) vers la chambre de fermentation ainsi que l'ouverture (15, 67) pour la conduite d'évacuation sont disposées aux extrémités opposées de la chambre de post-fermentation.

3. Installation suivant la revendication 1, caractérisée en ce que, dans la chambre de post-fermentation (4') près de l'ouverture d'entrée (19) du côté du fond, est prévue une plaque de paroi (222) qui relie partiellement la chambre de post-fermentation (4') avec le fond (2'), mais cependant ne s'étend que sur une partie de la hauteur intérieure de celle-ci.

4. Installation suivant l'une des revendications 1 ou 2, caractérisée en ce que la chambre de post-fermentation (4') est partagée par des plaques de paroi (22, 23) verticales se succédant en direction de la traversée, ces plaques étant alternativement reliées avec le fond (2') de la chambre de post-fermentation (4') et espacées de celui-ci en laissant une fente (24).

5. Installation suivant l'une quelconque des revendications 1 à 4, caractérisée en ce que l'ouverture (35, 54) de débouché tournée vers la chambre de fermentation (3', 3''', 3'v) de la conduite reliant la chambre de fermentation et la chambre de post-fermentation se trouve au-dessous du fond de la chambre de post-fermentation.

6. Installation suivant la revendication 1, caractérisé en ce que la chambre collectrice de gaz et la chambre de post-fermentation sont reliées entre elles par une conduite de prélèvement (25, 25''', 25'v) dont l'ouverture d'entrée est située dans la zone de fond de la chambre de post-fermentation et l'ouverture de sortie est située dans la zone supérieure de la chambre collectrice de gaz.

7. Installation suivant la revendication 6, caractérisée en ce que le conduit de prélèvement (25) s'étend, à partir de son orifice d'entrée (26) du côté du fond, tout d'abord, pour former un dispositif de fermeture hydraulique (27), en forme de courbe vers le bas.

8. Installation suivant la revendication 6, caractérisée en ce que la conduite de prélèvement (25''', 25'v) est disposée dans une chambre (37, 60) reliée à la chambre de post-fermentation (4''', 4'v), dont le fond (38, 55) se situe au-dessous du fond (2''', 2'v) de la chambre de post-fermentation (4''', 4'v).

9. Installation suivant l'une quelconque des revendications 1 à 8, caractérisée en ce que la chambre de fermentation (3') est formée par un cylindre dressé verticalement et la chambre de post-fermentation (4') est constituée par une chambre annulaire circulaire, située au-dessus, dont la partie centrale est reliée avec la chambre de fermentation et sert de chambre collectrice de gaz (7, 7'').

10. Installation suivant la revendication 1, caractérisée en ce que, au moins une partie de la conduite d'évacuation est isolée thermiquement et est en outre traversée par la conduite (9', 9'v) d'amenée de matière dans la chambre de fermentation (3', 3'v).

11. Installation suivant l'une des revendications 1 ou 10, caractérisée en ce que l'orifice d'entrée (15, 15') de la conduite d'évacuation (13, 13') hors de la chambre de post-fermentation (4, 4'), se trouve dans la zone supérieure de cette chambre, et effectivement à l'intérieur ou au-dessus du plan horizontal passant par le bord supérieur de débordement (16) du dispositif de fermeture hydraulique (14, 14').

12. Installation suivant la revendication 9, caractérisée en ce que la chambre de post-fermentation (4'') constituée comme un espace annulaire circulaire est partagée, par une cloison centrale (32), ces deux chambres qui sont parcourues par le liquide dans des directions de passage opposées.

13. Installation suivant l'une quelconque des revendications 1 à 12, caractérisée en ce que la chambre de fermentation (3, 3′′′, 3′ᵛ) et la chambre de post-fermentation (4, 4′′′, 4′ᵛ) sont séparées par un plafond intermédiaire (2′′′, 2′ᵛ) qui s'étend en position inclinée.

14. Installation suivant la revendication 1, caractérisée en ce que la chambre de fermentation (3′ᵛ) présente un second plafond (2′ᵛ) recouvrant la chambre de fermentation, s'étendant en face du plafond (48) du corps de construction (1′ᵛ) pour former une chambre collectrice de gaz (7′ᵛ) située plus bas, s'étendant obliquement, et se prolongeant par un dôme (59), et la partie de la chambre de post-fermentation (4′ᵛ) située latéralement à la chambre de fermentation (3′ᵛ) est reliée à celle-ci par une ouverture (54) proche du fond, la chambre de post-fermentation (4′ᵛ) étant munie d'une conduite d'évacuation (13′ᵛ) comportant une fermeture hydraulique du genre siphon, la chambre collectrice de gaz (7′ᵛ) étant reliée à un dispositif de soupape hydraulique (11′ᵛ), actionnable par la pression du gaz formé, pour transmettre suivant une cadence déterminée, le volume de gaz de la chambre collectrice (7′ᵛ) dans la chambre de post-fermentation (4′ᵛ) et simultanément pour évacuer par portion le schlamm en putréfaction, cette soupape (11′ᵛ) étant déclenchée par un levier (25′ᵛ).

15. Installation suivant la revendication 14, caractérisée en ce que l'ouverture voisine du fond (54) s'étend en forme de fente sur toute la largeur du corps de construction (1′ᵛ), un tablier horizontal (55) s'étendant, à partir du bord supérieur (53) de cette ouverture, vers la chambre de post-fermentation (4′ᵛ), une paroi séparatrice verticale (56) s'étendant, à partir du bord du tablier vers le plafond (48) du corps de construction (1′ᵛ), mais se terminant en laissant une fente de passage.

16. Installation suivant la revendication 15, caractérisée en ce que, dans une chambre (60) formée par la paroi séparatrice (56) et la paroi (52) qui limite la chambre de fermentation (3′ᵛ) par rapport à la chambre de post-fermentation (4′ᵛ), est disposé la conduite de prélèvement (25′ᵛ) qui s'étend jusqu'au tablier (55) et qui débouche dans le dôme (59) de la chambre collectrice de gaz (7′ᵛ).

17. Installation suivant l'une des revendications 15 ou 16, caractérisée en ce que le bord supérieur (58) de la cloison séparatrice (56) est situé à une faible distance au-dessus du bord de débordement de la conduite de prélèvement (25′ᵛ).

18. Installation suivant l'une quelconque des revendications 1 à 14, caractérisée en ce que la paroi transversale (47) qui limite la chambre de fermentation (3′ᵛ) dans le corps de construction (1′ᵛ) et qui présente l'ouverture d'introduction (49) pour les déchets à traiter, forme, avec une paroi frontale (46) du corps de construction, une chambre (61), qui est partagée, par au moins quatre parois séparatrices (62, 63, 64, 65) disposées de préférence symétriquement par rapport au plan médian du corps, les parois séparatrices situées à l'intérieur (62, 63) s'étendant jusqu'au fond (50) du corps de construction, pour former le puits de chargement (9′ᵛ), tandis que les deux parois séparatrices (64, 65) qui se trouvent à l'extérieur sont décalées du fond (50) du corps pour former des ouvertures de passage (67) pour le liquide.

19. Installation suivant la revendication 18, caractérisée en ce que les puits (68, 69) délimités par les parois séparatrices (64, 65) qui se trouvent à l'extérieur, débouchent, dans leur partie supérieure, dans la conduite d'évacuation (13′ᵛ) par l'intermédiaire de bords de débordement (70) réglables en hauteur.

20. Installation suivant la revendication 18, caractérisée en ce que les parois séparatrices situées à l'intérieur (62, 63) sont formées par des tôles métalliques judicieusement profilées.

21. Installation suivant la revendication 14, caractérisée en ce que l'installation de soupape (11′ᵛ) prévue dans le dôme (59) de la chambre collectrice de gaz (7′ᵛ) consiste en une chambre (82) en forme de pot, remplie en partie par un liquide (73), qui est reliée, par un tuyau (10′ᵛ) avec la chambre de post-fermentation (4′ᵛ), et en un tronçon de tuyau (74) en forme de U, disposé avec une de ses branches (75) dans la zone supérieure du dôme (59) et dont l'autre branche (77) s'avance dans la chambre en forme de pot (82), son orifice de débouché (84) se trouvant au-dessus du niveau du liquide, des perçages (78) étant prévus dans la portion de cette branche (77) qui est immergée dans le liquide (73).

22. Installation suivant la revendication 21, caractérisée en ce que la portion (76) du tronçon de tuyau en forme de U (74) qui réunit entre elles les deux branches (75, 77) s'étend horizontalement et est de préférence, plus longue que la distance entre les deux branches (75, 77).

23. Installation suivant l'une quelconque des revendications 1 à 22, caractérisée en ce que, dans le puits de remplissage (90), de préférence dans sa partie supérieure, sont prévus des bras (440) pouvant pivoter vers le bas à partir d'une position horizontale, qui s'étendent, dans leur position horizontale environ jusqu'au centre du puits de remplissage (90), plusieurs de ces bras étant disposés le long de la hauteur de puits.

Fig.1

Fig.5

0 013 538

Fig. 2

12'

15'  10'  11'  41  30  4'

29

25

13'

III                    7'  2'                        III

9'                                        26

31                          1'

18                    14'        27

3'

Fig. 3

1'

19

2'

23

222                    4'

7'

34                    22        21

18                    22        23

IV

9'        IV                    26

15'

28                    22

23        22

17

Fig. 4

## Fig.6

## Fig. 7

## Fig. 8

Fig. 9

Fig. 10

Fig. 11

Fig.16

Fig. 12

Fig. 18

Fig.13

Fig.17

Fig.14

Fig.15

## Fig.19